# EUROPÄISCHE PATENTSCHRIFT

(11) **EP 1 978 936 B1**
(45) Veröffentlichungstag und Bekanntmachung des Hinweises auf die Patenterteilung: **10.07.2013**
(21) Anmeldenummer: 07703962.6
(22) Anmeldetag: 17.01.2007
(51) Int. Cl.: A61K 9/16, A61K 9/20, A61K 9/50

(54) **Pharmazeutische Zusammensetzungen, enthaltend Mischungen aus Polymeren und in Wasser schlecht löslichen Wirkstoffen**
Pharmaceutical compositions containing mixtures of polymers and active agents poorly soluble in water
Compositions pharmaceutiques contenant des mélanges de polymères et d'agents actifs peu solubles dans l'eau

(30) Priorität: 03.02.2006 DE 102006005485
(43) Veröffentlichungstag der Anmeldung: 15.10.2008
(73) Patentinhaber: Evonik Röhm GmbH, 64293 Darmstadt (DE)
(72) Erfinder: MEIER, Christian, 64295 Darmstadt (DE); NOLLENBERGER, Kathrin, 60433 Frankfurt (DE); GRYCZKE, Andreas, 64560 Riedstadt (DE); PETEREIT, Hans-Ulrich, 64291 Darmstadt (DE); DRESSMAN, Jennifer, 60322 Frankfurt (DE)
(86) Internationale Anmeldenummer: PCT/EP2007/050465
(87) Internationale Veröffentlichungsnummer: WO 2007/090721

(56) Entgegenhaltungen:
- EP-A- 0 142 877
- EP-A- 1 064 935
- EP-A2- 0 682 945
- WO-A-03/000225
- WO-A1-2004/009059
- US-A1- 2004 071 773
- US-B1- 6 391 338

## Beschreibung

Die Erfindung betrifft verschiedene Pharmazeutische Zusammensetzungen, enthaltend Mischungen aus Polymeren und in Wasser schlecht löslichen Wirkstoffen

### Stand der Technik

EP 0 058 765 B1 beschreibt magensaftlösliche, quellbare Überzugsmassen und ihre Verwendung in einem Verfahren zum Überziehen von Arzneiformen. Es handelt sich insbesondere um wasserlösliche (Meth)acrylatcopolymere, die sich teilweise oder ganz aus Alkylacrylaten und/oder Alkylmethacrylaten mit einer tertiären Aminogruppe im Alkylrest zusammensetzen.

US 6,391,338 beschreibt die Löslichkeitsverbesserung bzw. Erhöhung der Bioverfügbarkeit von im wesentlichen wasserunlöslichen Wirkstoffen wie z. B. Ibuprofen, Itraconazol und Nifedipin mittels Flash Flow oder Extrusion der Wirkstoffe und Polymeren von Typ EUDRAGIT^{®} E. Während der Verarbeitung können dabei die Wirkstoffe in einen energetisch höheren Zustand übergehen (solid dispersion) und anschließend in Form von Nanopartikeln in einem gelösten Zustand freigesetzt werden.

US 6,319,520 beschreibt pharmazeutische Zusammensetzungen zur kontrollierten Wirkstofffreisetzung, bestehend aus thermisch formbaren Mischungen von mindestens einem Wirkstoff und einem oder mehreren pHunabhängigen Polymeren aus der Gruppe der Polymethacrylate. Die Herstellung der pharmazeutischen Zusammensetzungen kann mittels Spritzguss, Co-Spritzguss, Extrusion oder Coextrusion erfolgen. Bevorzugte (Meth)acrylatcopolymere sind EUDRAGIT® RL und RS, die gegebenenfalls auch zusammen mit EUDRAGIT^{®} E oder EUDRAGIT® L100, L100-55 und/oder S100 verwendet werden können. In den Beispielen werden u.a. die Wirkstoffe Benfluorex-Hydrochlorid, Ritmetidin-Dihydrogen, Fenspirid-Hydrochlorid mit EUDRAGIT® RL, RS und deren Mischungen mittels Extrusion oder Spritzguss verarbeitet.

WO 01/39751 A1 beschreibt ein Verfahren zur Herstellung von Formkörpern mittels Spritzguss. Die Verfahrensschritte umfassen
a) Aufschmelzen eines (Meth)acrylat-Copolymeren, das sich aus 30 bis 80 Gew.-% radikalisch polymerisierten C1- bis C4-Alkylestern der Acryl- oder der Methacrylsäure und 70 bis 20 Gew.-% (Meth)acrylat-Monomeren mit einer tertiären Ammoniumgruppe im Alkylrest zusammensetzt, wobei das (Meth)acrylat-Copolymere in Mischung mit 1 bis 70 Gew.-% von einem Weichmacher und einem Trockenstellmittel im Verhältnis 1: 1 bis 1 : 20 vorliegt, wobei mindestens 1 Gew.-% Weichmacher enthalten ist, sowie 0,05 bis 5 Gew.-% eines Trennmittels enthalten sind und zusätzlich weitere übliche Additive oder Hilfsstoffe und gegebenenfalls ein pharmazeutischer Wirkstoff in der Mischung enthalten sein können und die Mischung vor dem Aufschmelzen einen Gehalt an niedrigsiedenden Bestandteilen mit einem Dampfdruck von mindestens 1,9 bar bei 120°C von über 0,5 Gew.-% aufweist,
b) Entgasen der Mischung im thermoplastischen Zustand bei Temperaturen von mindestens 120 °C, wodurch der Gehalt der niedrigsiedenden Bestandteile mit einem Dampfdruck von mindestens 1,9 bar bei 120°C auf höchstens 0,5 Gew.-% gesenkt wird und
c) Einspritzen der aufgeschmolzenen und entgasten Mischung in den Formhohlraum eines Spritzgießwerkzeugs, wobei der Formhohlraum eine Temperatur aufweist, die mindestens 10 °C unterhalb der Glastemperatur des (Meth)acrylat-Copolymeren liegt, Abkühlen der Schmelzemischung und Entnahme des erhaltenen Formkörpers aus der Form.

Das (Meth)acrylatcopolymere, das bevorzugt ein EUDRAGIT® E sein kann, kann zur Steuerung der Wirkstoffabgabe in Mischung mit weiteren Polymeren vorliegen. Der Anteil weiterer Polymere soll nicht mehr als 20 Gew.-%, bevorzugt höchstens 10 Gew.-%, insbesondere 0-5 Gew.-% betragen. Als weitere Polymere für Mischungen werden u.a. EUDRAGIT® NE 30 D, EUDRAGIT® RS und EUDRAGIT® RL genannt. Das Verfahren kann auf beliebige Wirkstoffe angewendet werden, u.a. wird auch Ranitidin genannt.

WO 01/43935 A2 beschreibt ein Verfahren zur Herstellung von Formkörpern mittels Spritzguss mit den Verfahrensschritten
A) Aufschmelzen einer Mischung aus
   a) einem (Meth)acrylat-Copolymeren, das sich aus 40 bis 100 Gew.-% radikalisch polymerisierten C₁- bis C4-Alkylestern der Acryl- oder der Methacrylsäure und 0 bis 60 Gew.-% (Meth)acrylat-Monomeren mit einer anionischen Gruppe im Alkylrest zusammensetzt, das
   b) 0,1 bis 3 Gew.-% eines Trennmittels enthält
      und gegebenenfalls
   c) 0 bis 50 Gew.-% eines Trockenstellmittels
   d) 0 bis 30 Gew.-% eines Weichmachers
   e) 0 bis 100 Gew.-% Additive oder Hilfsstoffe
   f) 0 bis 100 Gew.-% eines pharmazeutischen-Wirkstoffs
   g) 0 bis 20 Gew.-% eines weiteren Polymeren oder Copolymeren in der Mischung enthalten sein können, wobei sich die Mengenangaben der Komponenten b) bis g) auf das (Meth)acrylat-Copolymere a) beziehen und
   die Mischung vor dem Aufschmelzen einen Gehalt an niedrigsiedenden Bestandteilen mit einem Dampfdruck von mindestens 1,9 bar bei 120°C von über 0,5 Gew.-% aufweist,
B) Entgasen der Mischung im thermoplastischen Zustand bei Temperaturen von mindestens 120 °C, wodurch der Gehalt der niedrigsiedenden Bestandteile mit einem Dampfdruck von mindestens 1,9 bar bei 120°C auf höchstens 0,5 Gew.-% gesenkt wird
C) Einspritzen der aufgeschmolzenen und entgasten Mischung in den Formhohlraum eines Spritzgießwerkzeugs, wobei der Formhohlraum eine Temperatur aufweist, die mindestens 10 °C unterhalb der Glastemperatur des (Meth)acrylat-Copolymeren liegt, Abkühlen der Schmelzemischung und Entnahme des erhaltenen Formkörpers aus der Form.

Die Mischung kann 0 bis 20 Gew.-% eines weiteren Polymeren oder Copolymeren g) enthalten. Zur Steuerung der Wirkstoffabgabe kann es im Einzelfall vorteilhaft sein, weitere Polymere zuzumischen. Der Anteil weiterer Polymere an der Mischung beträgt jedoch nicht mehr als 20 Gew.-%, bevorzugt höchstens 10 Gew.-%, insbesondere 0-5 Gew.-% .-%, bezogen auf das (Meth)acrylat-Copolymere, beträgt.

Beispiele für solche weiteren Polymere sind: Polyvinylpyrolidone, Polyvinylalkohole, kationische (Meth)acrylat-Copolymere aus Methylmethacrylat und/oder Ethylacrylat und 2-Dimethylaminoethylmethacrylat (EUDRAGIT^{®} E100), Carboxymethylcellulose-Salze, Hydroxypropylcellulose (HPMC), neutrale (Meth)acrylat Copolymere aus Methylmethacrylat und Ethylacrylat (Trockensubstanz aus EUDRAGIT^{®} NE 30 D), Copolymere aus Methylmethacrylat und Butylmethacrylat (PLASTOID^{®} B) oder (Meth)acrylat Copolymere mit quaternären Ammoniumgruppen, Trimethylammoniumethylmethacrylat-Chlorid als Monomer enthaltend (EUDRAGIT^{®} RL bzw. EUDRAGIT^{®} RS).

WO 2004/019918 beschreibt ein Verfahren zur Herstellung eines Granulats oder Pulvers, geeignet als Überzugs- und Bindemittel für orale oder dermale Arzneiformen, für Kosmetika oder Nahrungsergänzungsmittel, bestehend im wesentlichen aus (a) einem Copolymer, bestehend aus radikalisch polymerisierten C1- bis C4-Estern der Acryl- oder Methacrylsäure und weiteren (Meth)acrylat-Monomeren die funktionelle tertiäre Aminogruppen aufweisen, (b) 3 bis 25 Gew.-%, bezogen auf (a), eines Emulgators mit einem HLB-Wert von mindestens 14, (c) 5 bis 50 Gew.-%, bezogen auf (a), einer C₁₂- bis C₁₈-Monocarbonsäure oder einer C₁₂- bis C₁₈-Hydroxylverbindung, wobei die Komponenten (a), (b) und (c) gegebenenfalls unter Zusatz eines pharmazeutischen Wirkstoffs und/oder weiterer üblicher Zuschlagstoffe gleichzeitig oder nacheinander miteinander vermengt oder vermischt werden, in einem heizbaren Mischer geschmolzen, gemischt, die Schmelze abgekühlt und zu einem Granulat oder Pulver zerkleinert wird. Die verfahrensgemäß erhaltenen Granulate und Pulver eignen sich insbesondere zur Formulierung von feuchteempfindlichen pharmazeutischen Wirkstoffen, wie z.B. Acetylsalicyläure, Carbenoxolon, Cefalotin, Epinefrin, Imipramin, Kaliumjodid, Ketoprofen, Levodopa, Nitrazepam, Nitroprussid, Oxitetracyclin-HCl, Promethazin, Omeprazol oder andere Benzimidazolderivate, Ranitidin oder Streptomycin.

### Aufgabe und Lösung

US 6,391,338 beschreibt die Löslichkeitsverbesserung bzw. Erhöhung der Bioverfügbarkeit von im wesentlichen wasserunlöslichen Wirkstoffen wie z. B. Ibuprofen, Itraconazol und Nifedipin mittels Flash Flow oder Extrusion der Wirkstoffe und Polymeren von Typ EUDRAGIT® E. Während der Verarbeitung können dabei die Wirkstoffe in einen energetisch höheren Zustand übergehen (solid dispersion) und anschließend in Form von Nanopartikeln in einem gelösten Zustand freigesetzt werden. Es handelt sich um einen wissenschaftlich bemerkenswerten Ansatz, der in vielen Fällen zu guten Resultaten führt.

Gemäß der US 6,391,338 formulierte pharmazeutische Zusammensetzungen weisen in der Regel die Eigenschaft auf, einen enthaltenen Wirkstoff mit einer Löslichkeit in entmineralisiertem Wasser von 3,3 g/l oder weniger nach Auflösen einer EUDRAGIT® E-Matrix bei saurem pH in gelöster Form in einer Konzentration freizusetzen, die anfangs zunächst mindestens dem zweifachen Löslichkeitswert des Wirkstoffs in entmineralisiertem Wasser entspricht.

Die Erfinder haben jedoch festgestellt, dass dieser Effekt nur über einen relativ kurzen Zeitraum anhält. Nach dem anfänglichen Anstieg der Konzentration des messbar gelösten Wirkstoffs, fällt diese wieder unter die Grenze des zweifachen Löslichkeitswerts des Wirkstoffs in entmineralisiertem Wasser ab. Die Messung des gelösten Wirkstoffs kann dabei, je nach Wirkstofftyp oder Wirkstoffart, z. B. mittels chromatographischer oder spektrometrisch er Methoden, z. B. UV-Messung oder HPLC, oder mit anderen Methoden verfolgt werden. Die Erfinder vermuten, dass der zunächst höher energetische Zustand des Wirkstoffs sich nach dem Auflösen der EUDRAGIT^{®} E-Matrix rasch wieder zurückbildet und der Wirkstoff in eine schlecht- oder unlösliche Form, die dann auch nicht mehr oder bestenfalls eingeschränkt bioverfügbar ist, übergeht, eventuell sogar kristallisiert, aggregiert und/oder präzipitiert. Damit steht dieser Anteil des Wirkstoffs beim Übergang in das Duodenum nur noch bedingt zur Verfügung. Es besteht die Gefahr, dass die ursprünglich angestrebten Blutspiegelwerte nicht erreicht werden.

Bei in Wasser schlecht löslichen Wirkstoffen, die im Magen bzw. nach Magenpassage unmittelbar freigesetzt und resorbiert werden sollen, bei denen ein gewisser Blutspiegel für die therapeutische Wirkung erreicht werden muss, besteht somit das Problem, dass dieser oftmals nicht erreicht werden kann, da der Wirkstoff zu schnell wieder rekristallisiert oder präzipitiert und somit seine ursprüngliche erhöhte Bioverfügbarkeit wieder verloren geht.

WO 01/39751 A1 beschreibt ein Verfahren zur Herstellung von Formkörpern mittels Spritzguss. Insbesondere soll die Aufgabe gelöst werden, (Meth)acrylatcopolymere mit tertiären Aminogruppen in einer im Spritzguss verarbeitbaren Form bereitzustellen, so dass entsprechende Formteile in pharmazeutischer Qualität erhalten werden. Es wird erwähnt, dass neben (Meth)acrylatcopolymere mit tertiären Aminogruppen allein auch Mischungen mit EUDRAGIT® NE, EUDRAGIT® RS oder RL verarbeitet werden können. Beispiele zu solchen Mischungen allein oder in Kombination mit Wirkstoffen sind nicht enthalten. Ein Fachmann erhält aus WO 01/39751 A1 keine Hinweise zur Lösung des oben genannten Problems, eine länger anhaltende Löslichkeitsverbesserung für in Wasser schlecht lösliche Wirkstoffe zu bewirken.

WO 01/43935 A2 beschreibt ein Verfahren zur Herstellung von Formkörpern mittels Spritzguss. Insbesondere soll die Aufgabe gelöst werden,

(Meth)acrylatcopolymere mit anionischen Gruppen in einer im Spritzguss verarbeitbaren Form bereitzustellen, so dass entsprechende Formteile in pharmazeutischer Qualität erhalten. Es wird erwähnt, dass neben (Meth)acrylatcopolymere mit anionischen Gruppen allein auch Mischungen mit EUDRAGIT® NE, EUDRAGIT® RS oder RL verarbeitet werden können. Beispiele zu solchen Mischungen allein oder in Kombination mit Wirkstoffen sind nicht enthalten. Ein Fachmann erhält aus WO 01/43935 A2 keine Hinweise zur Lösung des oben genannten Problems, eine länger anhaltende Löslichkeitsverbesserung für in Wasser schlecht lösliche Wirkstoffe zu bewirken.

Ausgehend vom Stand der Technik sollte daher eine pharmazeutische Formulierung für in Wasser schlecht lösliche Wirkstoffe bereitgestellt werden, bei der eine gesteigerte Löslichkeit und damit verbundene Bioverfügbarkeit des Wirkstoffs in einer magensaftähnlichen Umgebung, pH 1,2, erreicht wird und über einen Zeitraum von mindestens 120 Minuten ganz oder zumindest teilweise stabil bleibt. Die magensaftähnliche Testumgebung stellt dabei die hohe Anforderung dar, so dass davon ausgegangen werden kann, dass der Zustand erhöhter Löslichkeit, wenn er in-vitro bei pH 1,2 nach 120 min stabil erreicht werden kann, sich auch in-vivo nach Übergang in den Darmabschnitt bei den dort herrschenden höheren pH-Werten nicht mehr wesentlich nachteilig verändert.

Die Aufgabe wird gelöst durch eine

Pharmazeutische Zusammensetzung, enthaltend eine Mischung aus zumindest einem kationischen, wasserlöslichen (Meth)acrylatcopolymer, zumindest einem wasserunlöslichen Polymer und zumindest einem Wirkstoff mit einer Löslichkeit in entmineralisiertem Wasser von 3,3 g/l oder weniger,
dadurch gekennzeichnet, dass
das wasserunlösliche Polymer und der Wirkstoff in einem Verhältnis von höchstens 3,5 zu 1 Gewichtsteilen vorliegen dass das oder gegebenenfalls die kationischen, wasserlöslichen (Meth) acrylatcopolymere und das oder gegebenenfalls die wasserunlöslichen Polymere in einem Verhältnis zueinander von 40 zu 60 bis 99 zu 1 Gewichtsteilen vorliegen, und die pharmazeutischen Zusammensetzung die Eigenschaft aufweist, den enthaltenen Wirkstoff in einem auf pH 1,2 gepufferten Medium in gelöster Form in einer Konzentration freizusetzen, die nach 2 Stunden bei pH 1,2 mindestens dem sechzehnfachen Löslichkeitswert des Wirkstoffs allein bei pH 1,2 entspricht, wobei das wasserunlösliche Polymer ein neutrales (Meth)acrylat-Copolymer, ein (Meth)acrylat-Copolymer mit quaternären Ammoniumgruppen, ein Polyvinylacetat oder ein Polyvinylacetat-Copolymer ist.

Die Erfindung betrifft weiterhin zwei alternative Verfahren zur Herstellung der erfindungsgemäßen pharmazeutischen Zusammensetzungen.

Die Erfindung betrifft weiterhin ein Verfahren zur Herstellung einer Arzneiform, enthaltend die erfindungsgemäßen pharmazeutische Zusammensetzung sowie die daraus resultierende Arzneiform.

Die Erfindung betrifft weiterhin die Verwendung der erfindungsgemäßen pharmazeutischen Zusammensetzungen zur Herstellung einer Arzneiform.

### Ausführung der Erfindung

Die Erfindung betrifft eine Pharmazeutische Zusammensetzung, vorzugsweise in Form eines Pulvers, enthaltend eine Mischung aus zumindest einem kationischen, wasserlöslichen (Meth)acrylatcopolymer, zumindest einem wasserunlöslichen Polymer und zumindest einem Wirkstoff mit einer Löslichkeit in entmineralisiertem Wasser von 3,3 g/l oder weniger,
dadurch gekennzeichnet, dass
das wasserunlösliche Polymer und der Wirkstoff in einem Verhältnis von höchstens 3,5 zu 1 Gewichtsteilen vorliegen dass das oder gegebenenfalls die kationischen, wasserlöslichen (Meth)acrylatcopolymere und das oder gegebenenfalls die wasserunlöslichen Polymere in einem Verhältnis zueinander von 40 zu 60 bis 99 zu 1 Gewichtsteilen vorliegen, und die pharmazeutische Zusammensetzung die Eigenschaft aufweist, den enthaltenen Wirkstoff in einem auf pH 1,2 gepufferten Medium (SGFsp, Simulated Gastric Fluid sine pancreatin) in gelöster Form in einer Konzentration freizusetzen, die nach 2 Stunden bei pH 1,2 mindestens dem sechzehnfachen Löslichkeitswert des Wirkstoffs allein bei pH 1,2 entspricht, wobei das wasserunlösliche Polymer ein neutrales (Meth)acrylat-Copolymer, ein (Meth)acrylat-Copolymer mit quaternären Ammoniumgruppen, ein Polyvinylacetat oder ein Polyvinylacetat-Copolymer ist.

### Kationische, wasserlösliche (Meth)acrylatcopolymere

Unter kationischen, wasserlöslichen (Meth)acrylatcopolymere werden solche. (Meth)acrylatcopolymere mit kationischen Gruppen verstanden, die zumindest in einem bestimmten pH-Bereich wasserlöslich sind. In der Regel ist in der pharmazeutischen Zusammensetzung nur ein kationisches, wasserlösliches (Meth)acrylatcopolymer enthalten. Es können jedoch gegebenenfalls auch zwei oder mehr kationische, wasserlösliche (Meth)acrylatcopolymere nebeneinander oder in Mischung vorliegen.

Das kationische, wasserlösliche (Meth)acrylatcopolymere hat möglicherweise die Funktion den schlecht in Wasser löslichen Wirkstoff im Falle einer Schmelzextrusion ähnlich zu US 6,391,338 in einen Zustand höherer Löslichkeit in der Polymermischung zu überführen.

Beispiele für bevorzugte kationische, wasserlösliche (Meth)acrylatcopolymere sind insbesondere (Meth)acrylat-Copolymere mit tertiären Aminogruppen:

### Typ EUDRAGIT® E

Das kationische, wasserlösliche (Meth)acrylatcopolymer kann sich teilweise oder ganz aus Alkylacrylaten und/oder Alkylmethacrylaten mit einer tertiären Aminogruppe im Alkylrest zusammensetzen. Geeignete (Meth)acrylatcopolymere sind bekannt z. B. aus EP 0 058 765 B1.

Das kationische, wasserlösliche (Meth)acrylat-Copolymer kann sich z.B. aus 30 bis 80 Gew.-% radikalisch polymerisierten C₁- bis C₄-Alkylestern der Acryl- oder der Methacrylsäure und 70 bis 20 Gew.-% (Meth)acrylat-Monomeren mit einer tertiären Aminogruppe im Alkylrest zusammensetzen.

Geeignete Monomere mit funktionellen tertiären Aminogruppen sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethly)propylacrylat, Dimethylamino-2,2-dimethly)propylmethacrylat, (3-Diethylamino-2,2-dimethly)propylacrylat und Diethylamino-2,2-dimethly)propylmethacrylat. Besonders bevorzugt ist Dimethylaminoethylmethacrylat.

Der Gehalt der Monomere mit tertiären Aminogruppen im Copolymeren kann vorteilhafterweise zwischen 20 und 70 Gew.-%, bevorzugt zwischen 40 und 60 Gew.-% liegen. Der Anteile der C₁- bis C₄-Alkylestern der Acryl- oder Methacrylsäure beträgt 70 - 30 Gew.-%. Zu nennen sind Methylmethacrylat, Ethylmethacrylat, Butylmethacrylat, Methylacrylat, Ethylacrylat und Butylacrylat.

Ein geeignetes (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein.

Ein konkret geeignetes handelsübliches (Meth)acrylatcopolymer mit tertiären Aminogruppen ist z. B. aus 25 Gew.-% Methylmethacrylat, 25 Gew.-% Butylmethacrylat und 50 Gew.-% Dimethylaminoethylmethacrylat aufgebaut (EUDRAGIT® E100 bzw. EUDRAGIT® E PO (Pulverform)). EUDRAGIT® E100 bzw. EUDRAGIT® E PO sind unterhalb von ca. pH 5,0 wasserlöslich und somit auch magensaftlöslich.

### Wasserunlösliche Polymere

Unter wasserunlöslichen Polymeren werden solche Polymere verstanden, die über den gesamten pH-Bereich von 1 bis 14 wasserunlöslich bzw. lediglich in Wasser quellbar sind. In der Regel ist in der pharmazeutischen Zusammensetzung nur ein wasserunlösliches Polymer enthalten. Es können jedoch gegebenenfalls auch zwei oder mehr wasserunlösliche Polymere nebeneinander oder in Mischung vorliegen.

Das wasserunlösliche Polymer hat vermutlich die Funktion den schlecht in Wasser löslichen Wirkstoff nach der Freisetzung aus der Arzneiform über einen längeren Zeitraum im Zustand höherer Löslichkeit zu stabilisieren und so eine löslichkeitvermindernde Aggregation, Rekristallisation oder Präzipitation zu verlangsamen oder zu verhindern.

Beispiele für bevorzugte wasserunlösliche Polymere sind insbesondere neutrale (Meth)acrylat-Copolymere sowie (Meth)acrylat-Copolymere mit quaternären Ammoniumgruppen:

### Neutrale (Meth)acrylatcopolymere (Typ EUDRAGIT^{®} NE oder Typ Eudragit® NM)

Neutrale oder im wesentlichen neutrale Methacrylat-Copolymere bestehen mindestens zu 95, insbesondere zu mindestens 98, bevorzugt zu mindestens 99, insbesondere zu mindestens 99, besonders bevorzugt zu 100 Gew.-% aus (Meth)acrylat-Monomeren mit neutralen Resten, insbesondere C₁- bis C₄-Alkylresten.

Geeignete (Meth)acrylat-Monomere mit neutralen Resten sind z. B. Methylmethacrylat, Ethylmethacrylat, Butylmethacrylat, Methylacrylat, Ethylacrylat, Butylacrylat. Bevorzugt sind Methylmethacrylat, Ethylacrylat und Methylacrylat.

In geringen Anteilen, zu weniger als 5, bevorzugt höchstens 2, besonders bevorzugt höchstens 1 oder 0,05 bis 1 Gew.-% können Methacrylatmonomere mit anionischen Resten, z. B. Acrylsäure und/oder Methacrylsäure, enthalten sein.

Geeignet sind z. B. neutrale oder nahezu neutrale (Meth)acrylat Copolymere aus 20 bis 40 Gew.-% Ethylacrylat, 60 bis 80 Gew.-% Methylmethacrylat und 0 bis weniger als 5, bevorzugt 0 bis 2 oder 0,05 bis 1 Gew.-% (Typ EUDRAGIT® NE).

EUDRAGIT® NE und Eudragit® NM sind Copolymere aus 30 Gew.-% Ethylacrylat und 70 Gew.-% Methylmethacrylat.

Bevorzugt sind neutrale oder im Wesentlichen neutrale Methylacrylatcopolymere, die gemäß der WO 01/68767 als Dispersionen unter Verwendung von 1-10 Gew.-% eines nichtionischen Emulgators mit einem HLB-Wert von 15, 2 bis 17,3 hergestellt wurden. Letztere bieten den Vorteil, dass eine Phasenseparation unter Ausbildung von Kristallstrukturen durch den Emulgator unterbleibt (Eudragit® NM).

Gemäß EP 1 571 164 A2 können entsprechende, nahezu neutrale (Meth)acrylatcopolymere, mit geringen Anteilen, 0,05 bis 1 Gew.-% an monoolefinisch ungesättigten C3-C8-Carbonsäuren jedoch auch durch Emulsionspolymerisation in Gegenwart vergleichsweise geringer Mengen anionischer Emulgatoren, z. B. 0,001 bis 1 Gew.-% hergestellt werden.

### (Meth)acrylatcopolymere mit quaternären Ammoniumgruppen (Typ EUDRAGIT® RS/RL)

Weitere geeignete, wasserunlösliche (Meth)acrylat-Copolymere sind z. B. aus EP-A 181 515 oder aus DE-PS 1 617 751 bekannt. Es handelt sich um unabhängig vom pH-Wert wasserunlösliche bzw. in Wasser lediglich quellbare Polymerisate, die für Arzneimittelüberzüge geeignet sind. Als mögliches Herstellungsverfahren ist die Substanzpolymeriation in Gegenwart eines im Monomerengemisch gelösten radikalbildenden Initiators zu nennen. Ebenso kann das Polymerisat auch mittels Lösungs-oder Fällungspolymerisation hergestellt werden. Das Polymerisat kann auf diese Weise in Form eines feinen Pulvers erhalten werden, was bei der Subtanzpolymerisation durch Mahlen, bei Lösungs- und Fällungspolymerisation z. B. durch Sprühtrocknung erreichbar ist.

Ein geeigentes wasserunlösliches (Meth)acrylat-Copolymer, setzt sich aus 85 bis 98 Gew.-% radikalisch polymerisierten C₁- bis C₄-Alkylestern der Acryl- oder der Methacrylsäure und 15 bis 2 Gew.-% (Meth)acrylat-Monomeren mit einer quaternären Aminogruppe im Alkylrest zusammen.

Bevorzugte C₁- bis C₄-Alkylestern der Acryl- oder der Methacrylsäure sind Methylacrylat, Ethylacrylat, Butylacrylat, Butylmethacrylat und Methylmethacrylat.

Als (Meth)acrylat Monomer mit quaternären Ammoniumgruppen wird 2-Trimethylammoniumethylmethacrylat-Chlorid besonders bevorzugt.

Ein entsprechendes Copolymer, kann z. B. aus 50 - 70 Gew.-% Methylmethacrylat, 20 - 40 Gew.-% Ethylacrylat und 7 - 2 Gew.-% 2-Trimethylammoniumethylmethacrylat-Chlorid aufgebaut sein.

Ein konkret geeignetes Copolymer enthält 65 Gew.-% Methylmethacrylat, 30 Gew.-% Ethylacrylat und 5 Gew.-% 2-Trimethylammoniumethylmethacrylat-Chlorid aufgebaut sein (EUDRAGIT® RS).

Ein weiteres geeignetes (Meth)acrylat-Copolymer kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylatMonomere sind handelsüblich und werden seit langem für retardierende Überzüge verwendet.

Ein konkret geeignetes Copolymer enthält z. B. 60 Gew.-% Methylmethacrylat, 30 Gew.-% Ethylacrylat und 10 Gew.-% 2-Trimethylammoniumethlymethacrylat-Chlorid (EUDRAGIT® RL).

Insbesondere kommen auch Mischungen der genannten (Meth)acrylatcopolymere in Frage, insbesondere Mischungen aus EUDRAGIT® RS und EUDRAGIT® RL z. B. im Verhältnis von 9 : 1 bis 1 : 9 Gewichtsteilen.

### Polyvinylacetat/Polyvinylacetat-Copolymere, Ethyl- und Methylcellulose

Die pharmazeutische Zusammensetzung kann als wasserunlösliches Polymer auch ein Polyvinylacetat, ein Polyvinylacetat-Copolymer (z. B. Typ Kollicoat® SR 30D oder Kollidon® SR), und, jedoch nicht als Teil der beanspruchten Erfindung, eine Ethylcellulose oder eine Methylcellulose enthalten.

### Mengenmäßige Anteile

Das oder gegebenenfalls die wasserlöslichen (Meth)acrylatcopolymere und das oder gegebenenfalls die wasserunlöslichen Polymere Liegen in der pharmazeutischen Zusammensetzung in einem Verhältnis zueinander von 40 zu 60 bis 99 zu 1, bevorzugt in einem Verhältnis zueinander von 50 zu 50 bis 95 zu 5, insbesondere in einem Verhältnis zueinander von 70 zu 30 bis 92 zu 8 Gewichtsteilen vor. Überraschender Weise genügen bereits geringe Beimengungen des wasserunlöslichen Polymeren zum wasserlöslichen (Meth)acrylatcopolymer, um den erfindungsgemäßen Effekt zu erreichen.

Der Anteil des wasserunlösliche Polymers, bezogen auf den Wirkstoff mit einer Löslichkeit in entmineralisiertem Wasser von 3,3 g/l oder weniger, soll nicht zu hoch sein, da sonst die angestrebte Löslichkeitsverbesserung nach 120 min bei pH 1,2 um mindestens das 16-fache nicht erreicht wird.

Das wasserunlösliche Polymer und der Wirkstoff sollen in einem Verhältnis von höchstens 3,5 Gewichtsteilen wasserunlöslichem Polymer zu 1 Gewichtsteil Wirkstoff, bevorzugt von höchstens 3,5 zu 1 bis 0,25 zu 1 Gewichtsteilen, insbesondere von höchstens 2,5 zu 1 bis 0,25 zu 1 Gewichtsteilen vorliegen. Im Falle des Vorliegens mehrerer wasserunlöslicher Polymere und/oder mehrerer Wirkstoffe nebeneinander, beziehen sich die Mengenanteile jeweils auf deren Summe.

### Wirkstoffe

Die pharmazeutische Zusammensetzung enthält zumindest einen, in der Regel nur einen, gegebenenfalls jedoch auch Kombinationen zweier oder mehrerer Wirkstoffe. Der enthaltene Wirkstoff kann daher aus einem einzigen oder gegebenenfalls auch aus mehreren einzelnen Wirkstoffen bestehen.

Der oder die Wirkstoffe weisen eine Löslichkeit in entmineralisiertem Wasser von 3,3 g/l oder weniger, bevorzugt 2,2 g/l oder weniger, insbesondere 1,1 g/l oder weniger auf.

Der oder die Wirkstoffe können z. B. der Gruppe der BCS-Klassen II und IV (Bio-Pharmaceutical-Classification-System nach Prof. Amidon; Amidon et al., Pharm. Res.12, 413 - 420 (1995)) und/oder aus der Gruppe der Antiandrogene, Antidepressiva, Antidiabetika, Antirheumatika, Glucocorticoide, Cytostatika, Migränemittel, Neuroleptika, Antibiotika, Östrogene, Vitamine, Psychopharmaca, ACE-Hemmer, β-Blocker, Ca-Kanalblocker, Diuretika, Herzglykoside, Antiepileptika, Diuretika/Antiglaukom, Urikostatika, H₂-Rezeptorenblocker und Virostatika angehören.

Die Wirkstoffe der BCS-Klasse II und IV weisen eine Löslichkeit in entmineralisiertem Wasser von 3,3 g/l oder weniger auf. Die Wirkstoffe der BCS-Klasse II sind gut permeabel, die der BCS-Klasse IV schlecht permeabel. Die erfindungsgemäßen Vorteile entfalten sich daher insbesondere bei den Wirkstoffen der BCS-Klasse II, da hier die Verfügbarkeit des Wirkstoffs in Lösung die einzige Limitierung für dessen Bioverfügbarkeit darstellt. Allerdings kann auch bei Wirkstoffen der BCS-Klasse IV eine erhöhte Verfügbarkeit des Wirkstoffs in Lösung hilfreich sein, um trotz der Limitierung der schlechten Aufnahme in die Zellen (Permeabilität) dieser Wirkstoffe zumindest graduell eine gewisse Verbesserung in der Bioverfügbarkeit zu erzielen.

Es kann bzw. können z. B. der oder gegebenenfalls die Wirkstoffe Bicalutamid, Anastrozol, Albendazol, Amitryptilin, Artemether, Chlorpromazin, Ciprofloxacin, Chlofazimin, Dapsone, Diloxanid, Efavirenz, Folsäure, Furosemid, Glibenclamid, Griseofulvin, Haloperidol, Ivermectin, Ibuprofen, Idinavir, Lopinavir, Lumefantrin, Mebendazol, Mefloquin, Niclosamid, Nelfinavir, Nifedipin, Nitrofurantoin, Phenytoin, Pyrantel, Pyremethamin, Retinol, Ritonavir, Spironolacton, Sulfadiacin, Sulfasalazin, Sulfamethoxazol, Triclabendazol, Trimethoprim, Valproinsäure, Verapamil, Warfarin, Nalidixinsäure, Nevirapin, Praziquantel, Rifampicin, Glimiprid, Nilutamid, Bromocriptin, Ketotifen, Letrozol, Naratriptan, Ganciclovir, Orlistat, Mesoprosztol, Granistron, Pioglitazon, Lamivudin, Rosiglitazon, Zidovudin, Enalapril, Atenolol, Nadolol, Felodipin, Bepridil, Digoxin, Digitoxin, Carbamazepin, Acetazolamid, Allopurinol, Cimetidin, Ranitidin oder Oxcarbazepin enthalten sein.

### Löslichkeit in Wasser

Die Erfindung betrifft Wirkstoffe mit einer Löslichkeit in entmineralisiertem Wasser von 3,3 g/l oder weniger, bevorzugt 3,3 g/l oder weniger, insbesondere 1,1 g/l oder weniger.

Die Löslichkeit in Wasser für den Wirkstoff kann nach DAB 10 (Deutsches Arzneibuch, 10. Ausgabe mit 3. Nachtrag 1994, Deutscher Apothekerverlag, Stuttgart und Govi Verlag, Frankfurt a. M., 2. Nachtrag (1993), IV Allgemeine Vorschriften, S. 5 - 6, "Löslichkeit und Lösungsmittel"; s. a. Ph. Eur. 4.07, 2004), definiert werden.

### Löslichkeit bei pH 1,2

Die Löslichkeit bei pH 1,2, bzw. die Menge des in gelöster Form vorliegenden Wirkstoffs, kann in einem nach USP (Paddle-Methode, 100 Upm) auf pH 1,2 gepufferten Medium (SGFsp, Simulated Gastric Fluid sine pancreatin) z.B. chromatographisch und/oder spektrometrisch bestimmt werden. Es werden die Werte des erfindungsgemäß formulierten Wirkstoffs und des nicht formulierten Wirkstoffs nach 120 min verglichen. Dies simuliert die Bedingungen einer durchschnittlichen Magenpassagezeit. In diesem Vergleich soll die Löslichkeit des erfindungsgemäß formulierten Wirkstoffs um mindestens das 16-fache, bevorzugt um mindestens das 18-fache, insbesondere um mindestens das 20-fache erhöht sein.

Dem Fachmann ist die Methodik nach USP, Paddle-Methode hinlänglich bekannt (s. z. B. USP 28-NF23, General Chapter <711>, *Dissolution,* Apparatus 2 (Paddle), Method <724> "Delayed Release (Enteric Coated) Articles-General General Drug Release Standard", Method B (100 Upm, 37 °C)).

### Verfahren zur Herstellung der pharmazeutischen Zusammensetzung

### "Lösungsmittelverfahren"

Die Erfindung betrifft weiterhin ein

Verfahren zur Herstellung einer pharmazeutischen Zusammensetzung in Form eines Feststoffs mit der Eigenschaft, den enthaltenen Wirkstoff in einem auf pH 1,2 gepufferten Medium in gelöster Form in einer Konzentration freizusetzen, die nach 2 Stunden bei pH 1,2 mindestens dem sechzehnfachen Löslichkeitswert des Wirkstoffs allein bei pH 1,2 entspricht, dadurch gekennzeichnet, dass zunächst eine Lösung in einem organischen Lösungsmittel oder einem Lösungsmittelgemisch aus dem kationischen, wasserlöslichen (Meth)acrylatcopolymer, dem Wirkstoff und dem wasserunlöslichen Polymer erzeugt wird, das Lösungsmittel anschließend z. B. durch Verdampfen oder Anlegen von Unterdruck, z. B. durch Gefriertrocknung oder Sprühtrocknung entfernt wird, wodurch ein Feststoff mit den genannten Eigenschaften erhalten wird.

Das organische Lösungsmittel kann gegebenenfalls auch ein Lösungsmittelgemisch mit anderen organischen Lösungsmitteln und/oder Wasser sein. Sofern Wasser enthalten ist, darf der Anteil nur so hoch sein, dass trotzdem noch alle Bestandteile, die beiden Polymertypen und der Wirkstoff, in eine Lösung übergehen. Geeignete Lösungsmittel sind z. B. Aceton, Isopropanol oder Ethanol oder Gemische davon. Geeignet ist z. B eine Mischung Isopropanol/Aceton mit 6 zu 4 Gewichtsteilen. Geeignet sind auch z. B. Ethanol/Wasser Mischungen, bevorzugt mit nicht mehr als 50 Gew.-% Wasser.

Das Verfahren macht sich zu Nutzen, dass der Wirkstoff in Wasser schlecht löslich ist und deshalb in einem organischen Lösungsmittel vergleichsweise gut gelöst werden kann. Die kationischen, wasserlöslichen (Meth)acrylatcopolymere sind zugleich auch in einem organischen Lösungsmittel lösbar. In geeigneter Form handelsüblich sind z. B. die Polymere von Typ EUDRAGIT® E auch in Form organischer Lösungen mit 12,5 % Feststoffgehalt. Das wasserunlösliche Polymer ist wiederum leicht in einem organischen Lösungsmittel löslich. Es ist daher möglich eine Lösung aller drei Komponenten herzustellen, wobei der Wirkstoff den gelösten Zustand auch nach dem Entfernen des Lösungsmittels im Feststoff bewahrt. Der Gehalt des wasserunlöslichen Polymers in der Mischung bewirkt aus unbekannten Gründen, dass die ursprüngliche vorhandene Löslichkeit des Wirkstoffs nach der Freisetzung in einem darmsaftähnlichem Medium bei pH 7,2 sich nicht wieder unter den Schwellenwert, der nach 4 Stunden mindestens dem zweifachen Löslichkeitswert des Wirkstoffs in entmineralisiertem Wasser entspricht, abfällt.

Das Lösungsmittelverfahren hat den Vorteil einer einfachen Ausführbarkeit.

### "Schmelzextrusionsverfahren"

Gegenüber dem Lösungsmittelverfahren ist das Schmelzextrusionsverfahren bevorzugt, u.a. da der aus Arbeits-, Gesundschutz- und aus Umweltschutzgründen problematische Umgang mit Lösungsmittel entfällt.

Die Erfindung betrifft erfindungsgemäß ein

Verfahren zur Herstellung einer pharmazeutischen Zusammensetzung in Form eines Extrudats mit der Eigenschaft aufweist, den enthaltenen Wirkstoff in einem auf pH 1,2 gepufferten Medium in gelöster Form in einer Konzentration freizusetzen, die nach 2 Stunden bei pH 1,2 mindestens dem sechzehnfachen Löslichkeitswert des Wirkstoffs allein bei pH 1,2 entspricht, dadurch gekennzeichnet, dass das kationische, wasserlösliche (Meth)acrylatcopolymer, der Wirkstoff und das wasserunlösliche Polymer gemischt und bei einer Temperatur im Bereich von 60 bis 220 °C, bevorzugt von 80 bis 180 °C, schmelzextrudiert werden.

Das Schmelzextrusionsverfahren kann mit Hilfe eines Extruders, insbesondere mittels eines Doppelschneckenextruders ausgeführt werden. Günstig ist es, wenn der Extruder bzw. der Doppelschneckenextruder mit einer Entgasungszone ausgestattet ist. Das kationische, wasserlösliche und das wasserunlösliche Polymer können als Feststoff, als Polymerlösung oder als Polymerdispersion eingearbeitet werden. Der Zusatz des Wirkstoffs kann als Feststoff, als Lösung oder als Suspension erfolgen. Das Extrudat wird bevorzugt mittels Stranggranulation und Heißabschlag zu zylindrisch, länglichen Stranggranulaten oder durch Heißabschlag unter Kühlung, zu ausgerundeten Pellets verarbeitet. EP 1 563 987 A1 beschreibt eine geeignete Vorrichtung zur Herstellung gerundeter Pellets (Pelletizer). Granulate können bevorzugterweise zu Pulvern mit, z. B. mit Korngrößen von kleiner/gleich 1 mm, bevorzugt im Bereich von 50 bis 500 µm, vermahlen werden.

### Verfahren zur Herstellung einer Arzneiform

Die Erfindung betrifft weiterhin ein

Verfahren zur Herstellung einer erfindungsgemäßen Arzneiform, enthaltend eine erfindungsgemäße pharmazeutische Zusammensetzung, dadurch gekennzeichnet, dass man eine pharmazeutische Zusammensetzung nach dem oben beschriebenen Lösungsmittelverfahren oder dem Schmelzextrusionsverfahren herstellt, zu Granulaten, Pellets oder Pulvern weiterverarbeitet und gegebenenfalls mittels pharmazeutisch üblicher Hilfsstoffen formuliert und in an sich bekannter Weise, z. B. durch Mischen, Verpressen, Powder Layering und/oder Verkapseln zu einer Arzneiform, z. B. zu Tabletten, oder bevorzugt zu einer multipartikulären Arzneiform, insbesondere zu pellethaltigen Tabletten, Minitabletten, Kapseln, Sachets oder Trockensäften, verarbeitet.

### Herstellung von Tabletten und multipartikulärer Arzneiformen

Die erfindungsgemäße pharmazeutische Zusammensetzung eignet sich insbesondere zur Herstellung von Arzneiformen in Tablettenform und zum Einsatz in multipartikulären Arzneiformen. Bevorzugt liegt die erfindungsgemäße pharmazeutische Zusammensetzung in Form eines Pulvers vor und kann direkt in praktisch allen bekannten pharmazeutischen Formulierungen, in denen der Wirkstoff in Pulverform eingearbeitet wird, anstelle des Wirkstoffs verwendet werden. Auf diese Weise können z. B., wie in WO 01/68058 oder WO 2005/046649, Neutralkerne (Non-Pareilles) im Powder-Layering-Verfahren mit der pharmazeutischen Zusammensetzung in Pulverform und einem Bindemittel beschichtet werden. Anschließend werden die beschichteten Kerne mit weiteren Hilfsstoffen und Polymerschichten, wie in WO 01/68058 oder WO 2005/046649 vorgegeben, zu fertigen Arzneiformen formuliert.

Für multipartikuläre Arzneiform kann die pharmazeutische Zusammensetzung in Form eines Pulvers auch ohne Neutralkern mit Bindemitteln durch Ausrunden, Verpressen zu wirkstoffhaltigen Partikeln oder Pellets verarbeitet werden, die ihrerseits zur Steuerung der Wirkstoffabgabe mit entsprechenden polymeren Überzugsschichten versehen werden können.

Die Herstellung von multipartikulären Arzneiformen zu Tabletten durch Verpressen eines pharmazeutisch üblichen Bindemittels mit wirkstoffhaltigen Partikeln ist z. B. Beckert et al. (1996), "Compression of enteric-coated pellets to disintegrating tablets", International Journal of Pharmaceutics 143, S. 13 - 23, und in WO 96/01624 ausführlich beschrieben.

Filmüberzüge auf wirkstoffhaltige Pellets werden üblicherweise in Wirbelschichtgeräten aufgebracht. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Zwischen wirkstoffhaltigen und einer gegebenenfalls vorhandenen darmsaftlöslichen Copolymer-Schicht kann eine trennende Schicht aufgebracht sein, die der Trennung von Wirkstoff und Überzugsmaterial zum Zwecke der Verhinderung von Interaktionen dient. Diese Schicht kann aus inerten Filmbildnern (z.B. HPMC oder HPC) oder z.B. Talkum oder anderen geeigneten pharmazeutischen Substanzen bestehen. Ebenso können Kombinationen aus Filmbildnern und Talkum oder ähnlichen Stoffen verwendet werden.

Es ist auch möglich eine Trennschicht aus teilweise bzw. vollneutralisierten Copolymer-Dispersionen, die z.B. anionische (Meth)acrylatcopolymere enthalten können, aufzubringen.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikeln erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Presskräften im Bereich von 5 bis 40 kN, bevorzugt 10-20 kN. Die Tablettenpressen können mit Systemen zur externen Schmierung ausgestattet sein.

### Hilfsstoffe

Der erfindungsgemäßen Zusammensetzung werden bevorzugt bei der Herstellung der Granulate, Pellets oder Pulver in an sich bekannter Weise übliche Hilfs- oder Zuschlagstoffe hinzugefügt. Grundsätzlich müssen natürlich alle eingesetzten Hilfsstoffe toxikologisch unbedenklich und insbesondere in Arzneimitteln ohne Risiko für Patienten zu verwenden sein.

Einsatzmengen und Verwendung der üblichen Zuschlagstoffe in Arzneimittelüberzügen oder Beschichtungen sind dem Fachmann geläufig. Übliche Zuschlagstoffe können z. B. Trennmittel, Pigmente, Stabilisatoren, Antioxidantien, Porenbildner, Penetrationsförderer, Glanzmittel, Aromastoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.

### ● Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca -

Stearat, gemahlene Kieselsäure, Kaolin oder nicht ionische Emulgatoren mit einem HLB - Wert zwischen 3 und 8 eingesetzt. Übliche Einsatzmengen für Trennmittel liegen zwischen 0,5 bis 100 Gew.-% bezogen auf die Summe aus Wirkstoff, wasserlöslichem (Meth)acrylatcopolymer und wasserunlöslichem Polymer.

### ● Pigmente:

Die zu verwendenden Pigmente nicht toxisch und für pharmazeutische Zwecke geeignet. Siehe dazu z. B. auch: Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Geeigente Pigmente sind z. B. Aluminiumoxidpigmente oder Gelborange , Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin (E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C. I. 16185, FD&C Red 2), Brilliantsäuregrün (E 142, C.I. 44090, FD&C Green S).

Die angegebenen E-Nummern der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummern beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

### ● Weichmacher

Weitere Zuschlagstoffe können auch Weichmacher sein. Übliche Mengen liegen zwischen 0 und 50, bevorzugt 5 bis 20 Gew.-%.

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC), Acetyltriethylcitrat (ATEC) und Dibutylsebacat (DBS). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wässriger Lösung oder nach thermischer Vorbehandlung einer Mischung vorgenommen werden. Auch können Mischungen von Weichmachern eingesetzt werden.

### Arzneiform

Die Erfindung betrifft weiterhin eine Arzneiform, enthaltend eine erfindungsgemäße pharmazeutische Zusammensetzung.

### Verwendung

Die Erfindung betrifft weiterhin die Verwendung der erfindungsgemäßen pharmazeutischen Zusammensetzung zur Herstellung einer Arzneiform. Die pharmazeutische Zusammensetzung kann dabei bevorzugter Weise in Pulverform anstelle eines pulverförmigen Wirkstoffs eingearbeitet werden. In der erfindungsgemäßen Formulierung hat das Pulver die Eigenschaft, den enthaltenen Wirkstoff in einem auf pH 1,2 gepufferten Medium in gelöster Form in einer Konzentration freizusetzen, die nach 2 Stunden bei pH 1,2 mindestens dem sechzehnfachen Löslichkeitswert des Wirkstoffs allein bei pH 1,2 entspricht. Dadurch wird es möglich an sich schlecht lösliche Wirkstoffe in einem Zustand erhöhter Löslichkeit in Arzneiformen aller Art einzubringen.

### Vorteilhafte Wirkungen der Erfindung

Eine vorteilhafte Wirkung der erfindungsgemäßen pharmazeutischen Zusammensetzung besteht insbesondere darin, dass in Wasser schlecht lösliche Wirkstoffe in einen Zustand höherer Löslichkeit überführt werden, wobei dieser Zustand, in Abgrenzung zum Stand der Technik (s. Beispiel 1), über einen Zeitraum von 120 min bei pH 1,2 stabil erhalten bleibt. Der Zeitraum von 120 min bei pH 1,2 simuliert dabei eine durchschnittliche Magenpassagezeit. Auf diese Weise ist es möglich, nach anfänglicher erhöhter Löslichkeit des Wirkstoffs, dessen Rekristallisation im Laufe der Magenaufenthaltsdauer zu vermindern oder sogar zu verhindern. Dies erhöht die Bioverfügbarkeit in zeitlicher Hinsicht und insbesondere im Moment des Übergangs im den Darmtrakt erheblich.

Die magensaftähnliche Umgebung stellt dabei eine hohe Testanforderung dar, so dass davon ausgegangen werden kann, dass der Zustand erhöhter Löslichkeit, wenn er im Test bei pH 1,2 nach 120 min stabil erreicht wird, sich auch nach Übergang in den Darmabschnitt bei den dort herrschenden höheren pH-Werten nicht mehr wesentlich nachteilig verändert.

Die erfindungsgemäße pharmazeutische Zusammensetzung eignet sich daher nicht nur für Wirkstoffe, die im Magen freigesetzt werden sollen, sondern praktisch auch für alle anderen Arzneiformen, z. B. für magensaftresistent überzogene und/oder retardierend formulierte Arzneiformen, die den Wirkstoff erst im Darm freisetzen. Dadurch ist es besser als bisher möglich, auch bei in Wasser schlecht löslichen Wirkstoffen therapeutisch erforderliche, vergleichsweise hohe Blutspiegelwerte einzustellen und auch über längere Zeiträume aufrecht zu erhalten.

Die pharmazeutische Zusammensetzung liegt bevorzugt in Pulverform vor und kann praktisch in allen Formulierung, in denen der Wirkstoff in Pulverform verarbeitet wird an dessen Stelle verwendet werden. Aufgrund der erhöhten Löslichkeit werden auf diese Weise grundsätzlich neue Therapiemöglichkeiten eröffnet.

Die vorteilhaften Wirkungen der Erfindung können z. B. anhand der Beispiele erläutert werden.

### Beispiele

### A) Polymere

EUDRAGIT® E ist ein wasserlösliches Copolymer aus 25 Gew.-% Methylmethacrylat, 25 Gew.-% Butylmethacrylat und 50 Gew.-% Dimethylaminoethylmethacrylat
EUDRAGIT® NE ist ein wasserunlösliches Copolymer aus 30 Gew.-% Ethylacrylat und 70 Gew.-% Methylmethacrylat.
EUDRAGIT® RL ist ein wasserunlösliches Copolymer 60 Gew.-% Methylmethacrylat, 30 Gew.-% Ethylacrylat und 10 Gew.-% 2-Trimethylammoniumethlymethacrylat-Chlorid.
Kollicoat® SR ist ein wasserunlösliches Polymer (ein Polyvinylacetat-Copolymer)
Kollicoat® IR (ein Vinylacetat-Ethylenglykol-Blockcopolymer) ist ein wasserlösliches Polymer
PEG 6000: Polyethylenglykol 6000 (wasserlösliches Polymer)

### B) Herstellung der Extrudate für die Beispiele 1 bis 14

Hergestellt werden die Proben durch Schmelzextrusion auf einem Doppelschneckenextruder (Leistritz MICRO 18 GL 40 D Pharma). Die Temperatur wurde so gewählt das mindestens eine Zone über dem Schmelzpunkt des Wirkstoffes liegt. Die Extrusion wurde in einem Bereich zwischen 70-170°C durchgeführt.

Felodipin, das wasserlösliche (Meth)acrylatcopolymer EUDRAGIT® E und gegebenenfalls das "zweite" Polymer werden über Feststoff- bzw. Flüssigdosierer eindosiert, im Extruder vermischt, aufgeschmolzen und extrudiert. In den erfindungsgemäßen Beispielen ist das "zweite" Polymer wasserunlöslich und liegt in einem Verhältnis zum Wirkstoff von höchstens 3,5 zu 1 vor. Die Drehzahl betrug 200 - 250 rpm. Die erhaltene Schmelze wird über ein luftgekühltes Abzugsband abgezogen und anschließend in einem Stranggranulator zerkleinert. Anschließend wird das Granulat in einer Retsch Ultra Zentrifugalmühle mit einem 250 µm Siebeinsatz bei 6000 1/min gemahlen und anschließend (< 250 µm) gesiebt.

### Zusammensetzung der einzelnen Extrudate in Gew.-%

**Tabelle 1:**

| Beispiel | Felodipin | EUDRAGIT® E | EUDRAGIT® NE | Kollicoat® SR | EUDRAGIT® RL | PEG 6000 | Kollicoat® IR |
|---|---|---|---|---|---|---|---|
| 1 | 10,0 | 90,0 | | | | | |
| 2 | 9,5 | 85,7 | 4,8 | | | | |
| 3 | 9,1 | 81,8 | 9,1 | | | | |
| 4 | 8,3 | 75,0 | 16,7 | | | | |
| 5 | 7,1 | 64,3 | 28,6 | | | | |
| 6 | 6,3 | 56,3 | 37,5 | | | | |
| 7 | 9,1 | 81,8 | | 9,1 | | | |
| 8 | 8,3 | 75,0 | | 16,7 | | | |
| 9 | 9,1 | 81,8 | | | 9,1 | | |
| 10 | 8,3 | 75,0 | | | 16,7 | | |
| 11 | 9,1 | 81,8 | | | | 9,1 | |
| 12 | 8,3 | 75,0 | | | | 16,7 | |
| 13 | 9,1 | 81,8 | | | | | 9,1 |
| 14 | 8,3 | 75,0 | | | | | 16,7 |

| | | | | | | | |
|---|---|---|---|---|---|---|---|
| Erfindungsgemäß: Beispiele 2-4. 7 - 10 Nicht erfindungsgemäß: Beispiele 1, 5, 6, 11 - 14 | | | | | | | |

### Beispiele 1 bis 14

### Freisetzung des Wirkstoffs Felodipin aus den gemahlenen Granulaten:

Die Freisetzung des Wirkstoffs aus den gemahlenen Granulaten wurde in einer Paddle-Apparatur (DT 700 Dissolutiontester, Erweka) USP 26 Methode 2, ausgeführt. Die Proben wurden entsprechend zu jeweils 10mg Felodipin eingewogen. 500ml SGFsp (Simulated Gastric Fluid sine pancreatin, USP) pH 1,2 (37°C ± 0,5) wurden als Medium verwendet und die Rührgeschwindigkeit betrug 100 rpm. 5 ml Proben wurden in bestimmten Abständen gezogen, durch einen Membranfilter (Rezist® 30/0,45 µm PTFE,Schleicher&Schüll) filtriert, und mit Methanol 1:1 verdünnt. Die ersten 2 ml wurden verworfen. Das entnommene Volumen wurde mit frischem, temperiertem Medium ersetzt. Die freigesetzte Menge an Felodipin wurde mittels HPLC detektiert.

(Verwendete Säule: RP 18 (Lichrospher 100,5 µm, 125x4, Merck,) Fließmittel: Acetonitril:Methanol:Phosphatpuffer pH3, Flussrate: 1ml/min, Wellenlänge: 362 nm)

### Bestimmung der Löslichkeit von Felodipin bei pH 1,2:

Felodipin hat eine Löslichkeit in Wasser < 1 mg/l (0,0001 g/I). Die Löslichkeit für Felodipin wurde zum Vergleich in auf pH 1,2 gepufferten Medium (SGFsp pH 1,2) bestimmt. Dafür wurden 10 mg in 20 ml Medium für 24 Stunden auf einer Schüttelplatte ("orbital shaker") bei 37°C in Bewegung gehalten. Die Konzentration wurde mittels HPLC bestimmt.

Für Felodipin allein, ohne erfindungsgemäße Formulierung, wurde eine Löslichkeit von 0,5 mg/l bestimmt.

Die Tabellen zu den Beispielen 1 bis 14 geben die Löslichkeitswerte von Felodipin bei pH 1,2 im zeitlichen Verlauf wieder. Es wurden drei Parallelversuche (Vessel 1 bis 3) ausgeführt.

### Beispiel 1 (nicht erfindungsgemäß):

**Extrudat aus EUDRAGIT ® E und Felodipin 90/10 (nicht erfindungsgemäß)**

| Zeit | Vessel 1 (g/l) | Vessel 2 (g/l) | Vessel 3 (g/l) | Mittelwert |
|---|---|---|---|---|
| 0 | 0,0000 | 0,0000 | 0,0000 | 0,0000 |
| 5 | 0,0126 | 0,0142 | 0,0164 | 0,0144 |
| 10 | 0,0129 | 0,0139 | 0,0158 | 0,0142 |
| 15 | 0,0111 | 0,0123 | 0,0144 | 0,0126 |
| 30 | 0,0073 | 0,0086 | 0,0107 | 0,0089 |
| 45 | 0,0055 | 0,0077 | 0,0077 | 0,0068 |
| 60 | 0,0048 | 0,0051 | 0,0059 | 0,0052 |
| 90 | 0,0042 | 0,0046 | 0,0043 | 0,0044 |
| 120 | 0,0037 | 0,0037 | 0,0038 | 0,0037 |

Das Löslichkeitsmaximum wird hier nach 5 min erreicht, fällt danach jedoch stark ab.

### Beispiel 2:

**Extrudat aus Felodipin, EUDRAGIT ® E und EUDRAGIT® NE 9,5/85,7/4,8**

| Zeit | Vessel 1 (g/l) | Vessel 2 (g/l) | Vessel 3 (g/l) | Mittelwert |
|---|---|---|---|---|
| 0 | 0 | 0 | 0 | 0 |
| 5 | 0,0120 | 0,0160 | 0,0159 | 0,0146 |
| 10 | 0,0141 | 0,0163 | 0,0168 | 0,0157 |
| 15 | 0,0154 | 0,0163 | 0,0164 | 0,0160 |
| 30 | 0,0160 | 0,0162 | 0,0155 | 0,0159 |
| 45 | 0,0158 | 0,0156 | 0,0152 | 0,0155 |
| 60 | 0,0154 | 0,0167 | 0,0153 | 0,0158 |
| 90 | 0,0150 | 0,0152 | 0,0153 | 0,0151 |
| 120 | 0,0150 | 0,0145 | 0,0141 | 0,0146 |

### Beispiel 3:

**Extrudat aus Felodipin, EUDRAGIT ® E und EUDRAGIT® NE 9,1/81,8/9,1**

| Zeit | Vessel 1 (g/l) | Vessel 2 (g/l) | Vessel 3 (g/l) | Mittelwert |
|---|---|---|---|---|
| 0 | 0 | 0 | 0 | 0 |
| 5 | 0,0099 | 0,0113 | 0,0121 | 0,0111 |
| 10 | 0,0127 | 0,0122 | 0,0123 | 0,0124 |
| 15 | 0,0128 | 0,0119 | 0,0120 | 0,0122 |
| 30 | 0,0121 | 0,0117 | 0,0119 | 0,0119 |
| 45 | 0,0120 | 0,0115 | 0,0123 | 0,0119 |
| 60 | 0,0124 | 0,0114 | 0,0120 | 0,0119 |
| 90 | 0,0133 | 0,0114 | 0,0114 | 0,0120 |
| 120 | 0,0116 | 0,0112 | 0,0111 | 0,0113 |

### Beispiel 4:

**Extrudat aus Felodipin, EUDRAGIT ® E und EUDRAGIT® NE 8,3/75/16,7**

| Zeit | Vessel 1 (g/l) | Vessel 2 (g/l) | Vessel 3 (g/l) | Mittelwert |
|---|---|---|---|---|
| 0 | 0,0000 | 0,0000 | 0,0000 | 0,0000 |
| 5 | 0,0044 | 0,0087 | 0,0087 | 0,0072 |
| 10 | 0,0094 | 0,0097 | 0,0099 | 0,0096 |
| 15 | 0,0097 | 0,0096 | 0,0093 | 0,0095 |
| 30 | 0,0095 | 0,0093 | 0,0094 | 0,0094 |
| 45 | 0,0098 | 0,0092 | 0,0099 | 0,0096 |
| 60 | 0,0094 | 0,0094 | 0,0096 | 0,0095 |
| 90 | 0,0095 | 0,0094 | 0,0099 | 0,0096 |
| 120 | 0,0103 | 0,0097 | 0,0095 | 0,0098 |

### Beispiel 5 (nicht erfindungsgemäß)

Extrudat aus Felodipin, EUDRAGIT ® E und EUDRAGIT® NE 7,14/64,28/28,58

| Zeit | Vessel 1 (g/l) | Vessel 2 (g/l) | Vessel 3 (g/l) | Mittelwert (g/l) |
|---|---|---|---|---|
| 0 | 0 | 0 | 0 | 0 |
| 5 | 0,0028 | 0,0046 | 0,0041 | 0,0039 |
| 10 | 0,0043 | 0,0044 | 0,0037 | 0,0041 |
| 15 | 0,0044 | 0,0041 | 0,0051 | 0,0045 |
| 30 | 0,0053 | 0,0053 | 0,0055 | 0,0054 |
| 45 | 0,0056 | 0,0051 | 0,0058 | 0,0055 |
| 60 | 0,0053 | 0,0056 | 0,0060 | 0,0056 |
| 120 | 0,0046 | 0,0055 | 0,0050 | 0,0051 |

### Beispiel 6 (nicht erfindungsgemäß)

Extrudat aus Felodipin, EUDRAGIT ® E und EUDRAGIT® NE 6,25/56,25/37,5

| Zeit | Vessel 1 (g/l) | Vessel 2 (g/l) | Vessel 3 (g/l) | Mittelwert (g/l) |
|---|---|---|---|---|
| 0 | 0 | 0 | 0 | 0 |
| 5 | 0,0008 | 0,0024 | 0,0024 | 0,0019 |
| 10 | 0,0022 | 0,0028 | 0,0027 | 0,0026 |
| 15 | 0,0026 | 0,0032 | 0,0028 | 0,0029 |
| 30 | 0,0036 | 0,0033 | 0,0030 | 0,0033 |
| 45 | 0,0031 | 0,0034 | 0,0032 | 0,0033 |
| 60 | 0,0037 | 0,0038 | 0,0034 | 0,0036 |
| 120 | 0,0011 | 0,0023 | 0,0010 | 0,0015 |

### Beispiel 7:

**Extrudat aus Felodipin, EUDRAGIT ® E und Kollicoat® SR 9,1/81,8/9,1**

| Zeit | Vessel 1 (g/l) | Vessel 2 (g/l) | Vessel 3 (g/l) | Mittelwert (g/l) |
|---|---|---|---|---|
| 0 | 0,0000 | 0,0000 | 0,0000 | 0,0000 |
| 5 | 0,0090 | 0,0116 | 0,0116 | 0,0107 |
| 10 | 0,0109 | 0,0119 | 0,0126 | 0,0118 |
| 15 | 0,0113 | 0,0119 | 0,0122 | 0,0118 |
| 30 | 0,0114 | 0,0116 | 0,0112 | 0,0114 |
| 45 | 0,0113 | 0,0116 | 0,0118 | 0,0116 |
| 60 | 0,0116 | 0,0123 | 0,0117 | 0,0119 |
| 120 | 0,0110 | 0,0119 | 0,0117 | 0,0115 |

### Beispiel 8:

**Extrudat aus Felodipin, EUDRAGIT ® E und Kollicoat® SR 8,3/75/16,7**

| Zeit | Vessel 1 (g/l) | Vessel 2 (g/l) | Vessel 3 (g/l) | Mittelwert (g/l) |
|---|---|---|---|---|
| 0 | 0,0000 | 0,0000 | 0,0000 | 0,0000 |
| 5 | 0,0069 | 0,0102 | 0,0095 | 0,0088 |
| 10 | 0,0100 | 0,0100 | 0,0101 | 0,0100 |
| 15 | 0,0117 | 0,0094 | 0,0101 | 0,0104 |
| 30 | 0,0096 | 0,0095 | 0,0098 | 0,0096 |
| 45 | 0,0094 | 0,0096 | 0,0103 | 0,0098 |
| 60 | 0,0096 | 0,0097 | 0,0098 | 0,0097 |
| 120 | 0,0097 | 0,0092 | 0,0099 | 0,0096 |

### Beispiel 9:

**Extrudat mit Felodipin, EUDRAGIT®E und EUDRAGIT ® RL 9,1/81,8/9,1**

| Zeit | Vessel 1 (g/l) | Vessel 2 (g/l) | Vessel 3 (g/l) | Mittelwert (g/l) |
|---|---|---|---|---|
| 0 | 0 | 0 | 0 | 0 |
| 5 | 0,0112 | 0,0130 | 0,0131 | 0,0124 |
| 10 | 0,0128 | 0,0135 | 0,0139 | 0,0134 |
| 15 | 0,0136 | 0,0144 | 0,0140 | 0,0140 |
| 30 | 0,0135 | 0,0142 | 0,0138 | 0,0138 |
| 45 | 0,0134 | 0,0132 | 0,0139 | 0,0135 |
| 60 | 0,0130 | 0,0123 | 0,0130 | 0,0128 |
| 120 | 0,0117 | 0,0113 | 0,0121 | 0,0117 |

### Beispiel 10:

**Extrudat mit Felodipin, EUDRAGIT®E und EUDRAGIT ® RL 8,3/75/16,7**

| Zeit | Vessel 1 (g/l) | Vessel 2 (g/l) | Vessel 3 (g/l) | Mittelwert (g/l) |
|---|---|---|---|---|
| 0 | 0,0000 | 0,0000 | 0,0000 | 0,0000 |
| 5 | 0,0077 | 0,0087 | 0,0117 | 0,0093 |
| 10 | 0,0101 | 0,0092 | 0,0101 | 0,0098 |
| 15 | 0,0103 | 0,0103 | 0,0101 | 0,0102 |
| 30 | 0,0101 | 0,0105 | 0,0103 | 0,0103 |
| 45 | 0,0101 | 0,0098 | 0,0104 | 0,0101 |
| 60 | 0,0102 | 0,0100 | 0,0097 | 0,0100 |
| 120 | 0,0095 | 0,0097 | 0,0106 | 0,0099 |

### Beispiel 11 (nicht erfindungsgemäß)

**Extrudat mit Felodipin, EUDRAGIT® E und PEG 6000 9,1/81,8/9,1**

| Zeit | Vessel 1 (g/l) | Vessel 2 (g/l) | Vessel 3 (g/l) | Mittelwert (g/l) |
|---|---|---|---|---|
| 0 | 0 | 0 | 0 | 0 |
| 5 | 0,0125 | 0,0161 | 0,0161 | 0,0149 |
| 10 | 0,0144 | 0,0158 | 0,0158 | 0,0153 |
| 15 | 0,0139 | 0,0147 | 0,0147 | 0,0144 |
| 30 | 0,0100 | 0,0106 | 0,0106 | 0,0104 |
| 45 | 0,0075 | 0,0076 | 0,0076 | 0,0075 |
| 60 | 0,0052 | 0,0058 | 0,0058 | 0,0056 |
| 120 | 0,0038 | 0,0043 | 0,0043 | 0,0041 |

### Beispiel 12 (nicht erfindungsgemäß)

**Extrudat mit Felodipin, EUDRAGIT® E und PEG 6000 8,3/75/16,7**

| Zeit | Vessel 1 (g/l) | Vessel 2 (g/l) | Vessel 3 (g/l) | Mittelwert (g/l) |
|---|---|---|---|---|
| 0 | 0 | 0 | 0 | 0 |
| 5 | 0,0152 | 0,0168 | 0,0203 | 0,0174 |
| 10 | 0,0156 | 0,0157 | 0,0145 | 0,0153 |
| 15 | 0,0144 | 0,0146 | 0,0152 | 0,0147 |
| 30 | 0,0109 | 0,0105 | 0,0092 | 0,0102 |
| 45 | 0,0087 | 0,0082 | 0,0081 | 0,0083 |
| 60 | 0,0073 | 0,0074 | 0,0072 | 0,0073 |
| 120 | 0,0062 | 0,0063 | 0,0058 | 0,0061 |

### Beispiel 13 (nicht erfindungsgemäß)

**Extrudat mit Felodipin, EUDRAGIT®E, und Kollicoat® IR 9,1/81,8/9,1**

| Zeit | Vessel 1 (g/l) | Vessel 2 (g/l) | Vessel 3 (g/l) | Mittelwert (g/l) |
|---|---|---|---|---|
| 0 | 0 | 0 | 0 | 0 |
| 5 | 0,0135 | 0,0224 | 0,0164 | 0,0174 |
| 10 | 0,0157 | 0,0180 | 0,0159 | 0,0165 |
| 15 | 0,0166 | 0,0168 | 0,0162 | 0,0165 |
| 30 | 0,0148 | 0,0151 | 0,0147 | 0,0149 |
| 45 | 0,0122 | 0,0122 | 0,0117 | 0,0120 |
| 60 | 0,0100 | 0,0105 | 0,0103 | 0,0103 |
| 120 | 0,0068 | 0,0067 | 0,0072 | 0,0069 |

### Beispiel 14 (nicht erfindungsgemäß)

**Extrudat mit Felodipin, EUDRAGIT®E und Kollicoat® IR 8,3/75/16,7**

| Zeit | Vessel 1 (g/l) | Vessel 2 (g/l) | Vessel 3 (g/l) | Mittelwert (g/l) |
|---|---|---|---|---|
| 0 | 0 | 0 | 0 | 0 |
| 5 | 0,0104 | 0,0130 | 0,0140 | 0,0125 |
| 10 | 0,0130 | 0,0137 | 0,0139 | 0,0136 |
| 15 | 0,0137 | 0,0141 | 0,0138 | 0,0139 |
| 30 | 0,0134 | 0,0140 | 0,0138 | 0,0138 |
| 45 | 0,0111 | 0,0125 | 0,0134 | 0,0123 |
| 60 | 0,0095 | 0,0116 | 0,0115 | 0,0109 |
| 90 | 0,0072 | 0,0079 | 0,0096 | 0,0082 |
| 120 | 0,00674 | 0,0084 | 0,00768 | 0,0076 |

### Zusammenfassung der Beispiele 1 bis 14 in Bezug auf die Löslichkeitserhöhung von Felodipin bei pH 1,2 nach 120 min

Die Ergebnisse sind in unten stehender **Tabelle 2** zusammengefasst

**Tabelle 2**

| **Beispiel** | **Gelöster Wirkstoff [g/l] nach 120 min bei pH 1,2** | **Gewichtsteile wasserunlösliches Polymer zu 1 Gewichtsteil Wirkstoff** | **Konzentrationserhöhung (Vielfaches), bezogen auf Löslichkeit des Wirkstoffs allein** |
|---|---|---|---|
| Felodipin allein | 0,0005 | - | - |
| 1 | 0,0037 | - | 7,4 |
| **2** | **0,0146** | **0,5** | **29,2** |
| **3** | **0,0113** | **1** | **22,6** |
| **4** | **0,0098** | **2** | **19,6** |
| 5 | 0,0050 | 4 | 10,0 |
| 6 | 0,0015 | 6 | 3,0 |
| **7** | **0,0115** | **1** | **23,0** |
| **8** | **0,0096** | **2** | **19,2** |
| **9** | **0,0117** | **1** | **23,4** |
| **10** | **0,0099** | **2** | **19,8** |
| 11 | 0,0041 | 1*⁾ | 8,2 |
| 12 | 0,0061 | 2*⁾ | 12,2 |
| 13 | 0,0069 | 1*⁾ | 13,8 |
| 14 | 0,0076 | 2*⁾ | 15,2 |

| | | | |
|---|---|---|---|
| *⁾ Verhältnis zum "zweiten" Polymer angegeben, welches in Bsp. 11 - 14 jedoch wasserlöslich ist. | | | |

### Erfindungsgemäße Beispiele 2 - 4, 7 - 10:

Es wird in allen Beispielen eine Löslichkeitserhöhung nach 120 min bei pH 1,2 um mindestens dem 16-fachen Wert von Felodipin allein bei pH 1,2 festgestellt.

### Nicht erfindungsgemäße Beispiele 1, 5, 6, 11 - 14:

Beispiel 1: Ohne den erfindungsgemäßen Zusatz eines wasserunlöslichen Polymers fällt die anfänglich gute Löslichkeit (nach 5 min, s. die Einzelwerte von Beispiel 1) auf den Faktor 7,4 nach 120 min.
Beispiele 5 und 6: Setzt man das wasserunlösliche Polymer bezogen auf den Wirkstoff in einem Verhältnis von mehr als 3,5 zu 1 Gewichtsteilen ein, so liegt die Löslichkeitsverbesserung unter dem 16-fachen des Wirkstoffs allein
Beispiele 11 bis 14: Setzt man anstelle eines wasserunlöslichen Polymers ein wasserlösliches Polymer ein, so liegt die Löslichkeitsverbesserung unter dem 16-fachen des Wirkstoffs allein.

## Patentansprüche

1. Pharmazeutische Zusammensetzung, enthaltend eine Mischung aus zumindest einem kationischen, wasserlöslichen (Meth)acrylatcopolymer, zumindest einem wasserunlöslichen Polymer und zumindest einem Wirkstoff mit einer Löslichkeit in entmineralisiertem Wasser von 3,3 g/l oder weniger,
**dadurch gekennzeichnet, dass**
das wasserunlösliche Polymer und der Wirkstoff in einem Verhältnis von höchstens 3,5 zu 1 Gewichtsteilen vorliegen, **dass das oder gegebenenfalls die kationischen, wasserlöslichen (Meth)acrylatcopolymere und das oder gegebenenfalls die wasserunlöslichen Polymere in einem Verhältnis zueinander von 40 zu 60 bis 99 zu 1 Gewichtsteilen vorliegen** und die pharmazeutische Zusammensetzung die Eigenschaft aufweist, den enthaltenen Wirkstoff in einem auf pH 1,2 gepufferten Medium in gelöster Form in einer Konzentration freizusetzen, die nach 2 Stunden bei pH 1,2 mindestens dem sechzehnfachen Löslichkeitswert des Wirkstoffs allein bei pH 1,2 entspricht, **wobei das wasserunlösliche Polymer ein neutrales (Meth)acrylat-Copolymer, ein (Meth)acrylat-Copolymer mit quaternären Ammoniumgruppen, ein Polyvinylacetat oder ein Polyvinylacetat-Copolymer ist.**

2. Pharmazeutische Zusammensetzung nach Anspruch 1, **dadurch gekennzeichnet, dass** sich das kationische, wasserlösliche (Meth)acrylatcopolymer teilweise oder ganz aus Alkylacrylaten und/oder Alkylmethacrylaten mit einer tertiären Aminogruppe im Alkylrest zusammensetzt.

3. Pharmazeutische Zusammensetzung nach Anspruch 2, **dadurch gekennzeichnet, dass** das kationische, wasserlösliche (Meth)acrylatcopolymer ein Polymerisat aus 30 bis 80 Gew.-% C₁- bis C₄-Alkylestern der Acryl- oder der Methacrylsäure und 70 bis 20 Gew.-% (Meth)acrylat-Monomeren mit einer tertiären Aminogruppe im Alkylrest ist.

4. Pharmazeutische Zusammensetzung nach Anspruch 2 oder 3, **dadurch gekennzeichnet, dass** das wasserlösliche (Meth)acrylatcopolymer ein Polymerisat aus 20 - 30 Gew.-% Methylmethacrylat, 20 - 30 Gew.-% Butylmethacrylat und 60 - 40 Gew.-% Dimethylaminoethylmethacrylat ist.

5. Pharmazeutische Zusammensetzung nach einem oder mehreren der Ansprüche 1 bis 4, **dadurch gekennzeichnet, dass** das wasserunlösliche Polymer ein Polymerisat aus 98 bis 88 Gew.-% C₁- bis C₄-Alkylestern der Acryl- oder der Methacrylsäure und 2 bis 12 Gew.-% aus (Meth)acrylat Monomeren mit einer quaternären Aminogruppe ist.

6. Pharmazeutische Zusammensetzung nach einem oder mehreren der Ansprüche 1 bis 5, **dadurch gekennzeichnet, dass** das wasserunlösliche Polymer ein Copolymer aus 20 bis 40 Gew.-% Ethylacrylat und 60 bis 80 Gew.-% Methylmethacrylat und 0 bis weniger als 5 Gew.-% Acrylsäure und/oder Methacrylsäure ist.

7. Pharmazeutische Zusammensetzung nach einem oder mehreren der Ansprüche 1 bis 6, **dadurch gekennzeichnet, dass** der Wirkstoff aus der Gruppe der BCS-Klassen II und IV (Bio-Pharmaceutical-Classification-System nach Prof. Amidon) und/oder aus der Gruppe der Antiandrogene, Antidepressiva, Antidiabetika, Antirheumatika, Glucocorticoide, Cytostatika, Migränemittel, Neuroleptika, Antibiotika, Östrogene, Vitamine, Psychopharmaca, ACE-Hemmer, β-Blocker, Ca-Kanalblocker, Diuretika, Herzglykoside, Antiepileptika, Diuretika/Antiglaukom, Urikostatika, H₂-Rezeptorenblocker und Virostatika ausgewählt ist.

8. Pharmazeutische Zusammensetzung nach einem oder mehreren der Ansprüche 1 bis 7, **dadurch gekennzeichnet, dass** der Wirkstoff Bicalutamid, Anastrozol, Albendazol, Amitryptilin, Artemether, Chlorpromazin, Ciprofloxacin, Chlofazimin, Dapsone, Diloxanid, Efavirenz, Folsäure, Furosemid, Glibenclamid, Griseofulvin, Haloperidol, Ivermectin, Ibuprofen, Idinavir, Lopinavir, Lumefantrin, Mebendazol, Mefloquin, Niclosamid, Nelfinavir, Nifedipin, Nitrofurantoin, Phenytoin, Pyrantel, Pyremethamin, Retinol, Ritonavir, Spironolacton, Sulfadiacin, Sulfasalazin, Sulfamethoxazol, Triclabendazol, Trimethoprim, Valproinsäure, Verapamil, Warfarin, Nalidixinsäure, Nevirapin, Praziquantel, Rifampicin, Glimiprid, Nilutamid, Bromocriptin, Ketotifen, Letrozol, Naratriptan, Ganciclovir, Orlistat, Mesoprosztol, Granistron, Pioglitazon, Lamivudin, Rosiglitazon, Zidovudin, Enalapril, Atenolol, Nadolol, Felodipin, Bepridil, Digoxin, Digitoxin, Carbamazepin, Acetazolamid, Allopurinol, Cimetidin, Ranitidin oder Oxcarbazepin ist.

9. Pharmazeutische Zusammensetzung nach einem oder mehreren der Ansprüche 1 bis 8, **dadurch gekennzeichnet, dass** sie in Form eines Pulvers vorliegt.

10. Verfahren zur Herstellung einer pharmazeutischen Zusammensetzung nach einem oder mehreren der Ansprüche 1 bis 9 in Form eines granulierten oder gemahlenen Extrudats mit der Eigenschaft, den enthaltenen Wirkstoff in einem auf pH 1,2 gepufferten Medium in gelöster Form in einer Konzentration freizusetzen, die nach 2 Stunden bei pH 1,2 mindestens dem sechzehnfachen Löslichkeitswert des Wirkstoffs allein bei pH 1,2 entspricht, **dadurch gekennzeichnet, dass** das kationische, wasserlösliche (Meth)acrylatcopolymer, der Wirkstoff und das wasserunlösliche Polymer gemischt und bei einer Temperatur im Bereich von 60 bis 220 °C schmelzextrudiert werden und das Extrudat zu einen Granulat zerkleinert bzw. gemahlen wird.

11. Verfahren zur Herstellung einer pharmazeutischen Zusammensetzung nach einem oder mehreren der Ansprüche 1 bis 9 in Form eines Feststoffs mit der Eigenschaft, den enthaltenen Wirkstoff in einem auf pH 1,2 gepufferten Medium in gelöster Form in einer Konzentration freizusetzen, die nach 2 Stunden bei pH 1,2 mindestens dem sechzehnfachen Löslichkeitswert des Wirkstoffs allein bei pH 1,2 entspricht, **dadurch gekennzeichnet, dass** zunächst eine Lösung in einem organischen Lösungsmittel oder einem Lösungsmittelgemisch aus dem kationischen, wasserlöslichen (Meth)acrylatcopolymer, dem Wirkstoff und dem wasserunlöslichen Polymer erzeugt wird, das organische Lösungsmittel anschließend z. B. durch Verdampfen oder Anlegen von Unterdruck entfernt wird, wodurch ein Feststoff mit den genannten Eigenschaften erhalten wird.

12. Verfahren zur Herstellung einer Arzneiform, enthaltend eine pharmazeutische Zusammensetzung nach einem oder mehreren der Ansprüche 1 bis 9, **dadurch gekennzeichnet, dass** man eine pharmazeutische Zusammensetzung gemäß Anspruch 10 oder 11 herstellt, zu Granulaten, Pellets oder Pulvern weiterverarbeitet, gegebenenfalls mittels pharmazeutisch üblicher Hilfsstoffen formuliert und in an sich bekannter Weise, z. B. durch Mischen, Verpressen, Powder Layering und/oder Verkapseln zu einer Arzneiform, z. B. zu Tabletten, oder bevorzugt zu einer multipartikulären Arzneiform, insbesondere zu pellethaltigen Tabletten, Minitabletten, Kapseln, Sachets oder Trockensäften, verarbeitet.

13. Arzneiform, enthaltend eine pharmazeutische Zusammensetzung nach einem oder mehreren der Ansprüche 1 bis 9.

14. Verwendung einer pharmazeutischen Zusammensetzung nach einem oder mehreren der Ansprüche 1 bis 9 zur Herstellung einer Arzneiform.

## Claims

1. Pharmaceutical composition comprising a mixture of at least one cationic, water-soluble (meth)acrylate copolymer, at least one water-insoluble polymer and at least one active ingredient having a solubility in demineralized water of 3.3 g/l or less,
**characterized in that**
the water-insoluble polymer and the active ingredient are present in a ratio of at most 3.5 to 1 parts by weight, **in that** the cationic, water-soluble (meth)acrylate copolymer or copolymers and the water-insoluble polymer or polymers are present in a ratio relative to one another of 40:60 to 99:1 parts by weight,
and the pharmaceutical composition has the property of releasing the active ingredient present in a medium buffered to pH 1.2 in dissolved form in a concentration which, after 2 hours at pH 1.2, corresponds to at least sixteen times the solubility value of the active ingredient alone at pH 1.2 the water-insoluble polymer being a neutral (meth)acrylate copolymer, a meth(acrylate) copolymer having quaternary ammonium groups, a polyvinyl acetate or a polyvinyl acetate copolymer.

2. Pharmaceutical composition according to Claim 1, **characterized in that** the cationic, water-soluble (meth)acrylate copolymer is composed partly or fully of alkyl acrylates and/or alkyl methacrylates having a tertiary amino group in the alkyl radical.

3. Pharmaceutical composition according to Claim 2, **characterized in that** the cationic, water-soluble (meth)acrylate copolymer is an addition polymer composed of 30 to 80% by weight of C₁- to C₄-alkyl esters of acrylic or of methacrylic acid, and 70 to 20% by weight of (meth)acrylate monomers having a tertiary amino group in the alkyl radical.

4. Pharmaceutical composition according to Claim 2 or 3, **characterized in that** the water-soluble (meth)acrylate copolymer is an addition polymer composed of 20 - 30% by weight of methyl methacrylate, 20 - 30% by weight of butyl methacrylate and 60 - 40% by weight of dimethylaminoethyl methacrylate.

5. Pharmaceutical composition according to one or more of Claims 1 to 4, **characterized in that** that the water-insoluble polymer is an addition polymer composed of 98 to 88% by weight of C₁- to C₄-alkyl esters of acrylic acid or of methacrylic acid and 2 to 12% by weight of (meth)acrylate monomers having a quaternary amino group.

6. Pharmaceutical composition according to one or more of Claims 1 to 5, **characterized in that** the water-insoluble polymer is a copolymer composed of 20 to 40% by weight of ethyl acrylate and 60 to 80% by weight of methyl methacrylate and 0 to less than 5% by weight of acrylic acid and/or methacrylic acid.

7. Pharmaceutical composition according to one or more of Claims 1 to 6, **characterized in that** the active ingredient is selected from the group of BCS classes II and IV (Biopharmaceutical classification system according to Prof. Amidon) and/or from the group of the antiandrogenics, antidepressives, antidiabetics, antirheumatics, glucocorticoids, cytostatics, migraine drugs, neuroleptics, antibiotics, oestrogens, vitamins, psychotropic drugs, ACE inhibitors, β-blockers, calcium channel blockers, diuretics, cardiac glycosides, antiepileptics, diuretics/antiglaucoma, uricostatics, H₂ receptor blockers and virostatics.

8. Pharmaceutical composition according to one or more of Claims 1 to 7, **characterized in that** the active ingredient is bicalutamide, anastrozole, albendazole, amitryptiline, artemether, chlorpromazine, ciprofloxacin, clofazimine, dapsone, diloxanide, efavirenz, folic acid, furosemide, glibenclamide, griseofulvin, haloperidol, ivermectin, ibuprofen, idinavir, lopinavir, lumefantrin, mebendazole, mefloquin, niclosamide, nelfinavir, nifedipine, nitrofurantoin, phenytoin, pyrantel, pyremethamine, retinol, ritonavir, spironolactone, sulfadiazine, sulfasalazine, sulfamethoxazole, triclabendazole, trimethoprim, valproic acid, verapamil, warfarin, nalidixic acid, nevirapine, praziquantel, rifampicin, glimipiride, nilutamide, bromocriptine, ketotifen, letrozole, naratriptan, ganciclovir, orlistat, misoprostol, granistron, pioglitazone, lamivudine, rosiglitazone, zidovudine, enalapril, atenolol, nadolol, felodipine, bepridil, digoxin, digitoxin, carbamazepine, acetazolamide, allopurinol, cimetidine, ranitidine or oxcarbazepine.

9. Pharmaceutical composition according to one or more of Claims 1 to 8, **characterized in that** it is present in the form of a powder.

10. Process for preparing a pharmaceutical composition according to one or more of Claims 1 to 9 in the form of a granulated or ground extrudate with the property of releasing the active ingredient present in a medium buffered to pH 1.2 in dissolved form in a concentration which, after 2 hours at pH 1.2, corresponds to at least sixteen times the solubility value of the active ingredient alone at pH 1.2, **characterized in that** the cationic, water-soluble (meth)acrylate copolymer, the active ingredient and the water-insoluble polymer are mixed and melt-extruded at a temperature in the range of 60 to 220°C, and the extrudate is comminuted or ground to a granule.

11. Process for preparing a pharmaceutical composition according to one or more of Claims 1 to 9 in the form of a solid with the property of releasing the active ingredient present in a medium buffered to pH 1.2 in dissolved form in a concentration which, after 2 hours at pH 1.2, corresponds to at least sixteen times the solubility value of the active ingredient alone at pH 1.2, **characterized in that** a solution in an organic solvent or a solvent mixture composed of the cationic, water-soluble (meth)acrylate copolymer, the active ingredient and the water-insoluble polymer is first obtained, the organic solvent is then removed, for example, by evaporation or applying reduced pressure, which affords a solid with the named properties.

12. Process for producing a pharmaceutical form comprising a pharmaceutical composition according to one or more of Claims 1 to 9, **characterized in that** a pharmaceutical composition according to Claim 10 or 11 is prepared, processed further to granules, pellets or powders, if appropriate formulated by means of pharmaceutically customary excipients, and processed in a manner known per se, for example by mixing, compressing, powder layering and/or encapsulation to a pharmaceutical form, for example to tablets, or preferably to a multiparticulate pharmaceutical form, especially to pellet-containing tablets, minitablets, capsules, sachets or reconstitutable powders.

13. Pharmaceutical form comprising a pharmaceutical composition according to one or more of Claims 1 to 9.

14. Use of a pharmaceutical composition according to one or more of Claims 1 to 9 for producing a pharmaceutical form.

## Revendications

1. Composition pharmaceutique, contenant un mélange d'au moins un copolymère de (méth)acrylate cationique, hydrosoluble, au moins un polymère insoluble dans l'eau et au moins une substance active ayant une solubilité dans l'eau déminéralisée de 3,3 g/l ou moins,
**caractérisée en ce que**
le polymère insoluble dans l'eau et la substance active sont présents en un rapport d'au maximum 3,5 : 1 parties en poids, **en ce que** le ou éventuellement les copolymères de (méth)acrylate cationiques, hydrosolubles et le ou éventuellement les polymères insolubles dans l'eau sont présents en un rapport entre eux de 40 : 60 à 99 : 1 parties en poids et la composition pharmaceutique présente la propriété de libérer dans un milieu tamponné à pH 1,2 la substance active contenue, sous forme dissoute, à une concentration qui après 2 heures à pH 1,2 correspond à au moins seize fois l'indice de solubilité de la substance active seule à pH 1,2, le polymère insoluble dans l'eau étant un copolymère de (méth)acrylate neutre, un copolymère de (méth)acrylate à groupes ammonium quaternaire, un poly(acétate de vinyle) ou un copolymère de poly(acétate de vinyle).

2. Composition pharmaceutique selon la revendication 1, **caractérisée en ce que** le copolymère de (méth)acrylate cationique, hydrosoluble, est composé en partie ou en totalité d'acrylates d'alkyle et/ou de méthacrylates d'alkyle comportant un groupe amino tertiaire dans le radical alkyle.

3. Composition pharmaceutique selon la revendication 2, **caractérisée en ce que** le copolymère de (méth)acrylate cationique, hydrosoluble, est un polymérisat de 30 à 80 % en poids d'esters alkyliques en C₁-C₄ de l'acide acrylique ou de l'acide méthacrylique et de 70 à 20 % en poids de monomères (méth)acrylate comportant un groupe amino tertiaire dans le radical alkyle.

4. Composition pharmaceutique selon la revendication 2 ou 3, **caractérisée en ce que** le copolymère de (méth)acrylate hydrosoluble est un polymérisat de 20 - 30 % en poids de méthacrylate de méthyle, 20 - 30 % en poids de méthacrylate de butyle et 60 - 40 % en poids de méthacrylate de diméthyl-aminoéthyle.

5. Composition pharmaceutique selon une ou plusieurs des revendications 1 à 4, **caractérisée en ce que** le polymère insoluble dans l'eau est un polymérisat de 98 à 88 % en poids d'esters alkyliques en C₁-C₄ de l'acide acrylique ou de l'acide méthacrylique et de 2 à 12 % en poids de monomères (méth)acrylate comportant un groupe amino quaternaire.

6. Composition pharmaceutique selon une ou plusieurs des revendications 1 à 5, **caractérisée en ce que** le polymère insoluble dans l'eau est un copolymère de 20 à 40 % en poids d'acrylate d'éthyle et de 60 à 80 % en poids de méthacrylate de méthyle et de 0 à moins de 5 % en poids d'acide acrylique et/ou d'acide méthacrylique.

7. Composition pharmaceutique selon une ou plusieurs des revendications 1 à 6, **caractérisée en ce que** la substance active est choisie dans le groupe des classes BCS II et IV (Bio-pharmaceutical-Classification-System selon le Prof. Amidon) et/ou dans le groupe des antiandrogènes, antidépresseurs, antidiabétiques, antirhumatismaux, glucocorticoïdes, cytostatiques, antimigraineux, neuroleptiques, antibiotiques, oestrogènes, vitamines, psychotropes, inhibiteurs d'ACE, β-bloquants, inhibiteurs calciques, diurétiques, glycosides cardiotoniques, anti-épileptiques, diurétiques/ antiglaucomateux, uricostatiques, inhibiteurs des récepteurs H₂ et virostatiques.

8. Composition pharmaceutique selon une ou plusieurs des revendications 1 à 7, **caractérisée en ce que** la substance active est le bicalutamide, l'anastrozole, l'albendazole, l'amitryptiline, l'artéméther, la chlorpromazine, la ciprofloxacine, la chlofazimine, la dapsone, le diloxanide, l'éfavirenz, l'acide folique, le furosémide, le glibenclamide, la griséofulvine, l'halopéridol, l'ivermectine, l'ibuprofène, l'idinavir, le lopinavir, la luméfantrine, le mébendazole, la méfloquine, le niclosamide, le nelfinavir, la nifédipine, la nitrofurantoïne, la phénytoïne, le pyrantel, la pyréméthamine, le rétinol, le ritonavir, la spironolactone, la sulfadiacine, la sulfasalazine, le sulfaméthoxazole, le triclabendazole, le triméthoprim, l'acide valproïque, le vérapamil, la warfarine, l'acide nalidixique, la névirapine, le praziquantel, la rifampicine, le glimipride, le nilutamide, la bromocriptine, le kétotifène, le létrozole, le naratriptan, le ganciclovir, l'orlistat, le misoprostol, le granistron, la pioglitazone, la lamivudine, la rosiglitazone, la zidovudine, l'énalapril, l'aténolol, le nadolol, la félodipine, le bépridil, la digoxine, la digitoxine, la carbamazépine, l'acétazolamide, l'allopurinol, la cimétidine, la ranitidine ou l'oxcarbazépine.

9. Composition pharmaceutique selon une ou plusieurs des revendications 1 à 8, **caractérisée en ce qu'**elle se présente sous forme d'une poudre.

10. Procédé pour la préparation d'une composition pharmaceutique selon une ou plusieurs des revendications 1 à 9, sous forme d'un extrudat granulé ou broyé, ayant la propriété de libérer dans un milieu tamponné à pH 1,2 la substance active contenue, sous forme dissoute, à une concentration qui après 2 heures à pH 1,2 correspond à au moins seize fois l'indice de solubilité de la substance active seule à pH 1,2, **caractérisé en ce qu'**on mélange le copolymère de (méth)acrylate cationique, hydrosoluble, la substance active et le polymère insoluble dans l'eau et on extrude à chaud le mélange à une température dans la plage de 60 à 220 °C et on fragmente ou broie l'extrudat en un granulé.

11. Procédé pour la préparation d'une composition pharmaceutique selon une ou plusieurs des revendications 1 à 9, sous forme d'un solide ayant la propriété de libérer dans un milieu tamponné à pH 1,2 la substance active contenue, sous forme dissoute, à une concentration qui après 2 heures à pH 1,2 correspond à au moins seize fois l'indice de solubilité de la substance active seule à pH 1,2, **caractérisé en ce que** d'abord on produit une solution dans un solvant organique ou un mélange de solvants à partir du copolymère de (méth)acrylate cationique, hydrosoluble, de la substance active et du polymère insoluble dans l'eau, ensuite on élimine le solvant organique par exemple par évaporation ou application d'une dépression, de manière à obtenir un solide ayant les propriétés indiquées.

12. Procédé pour la fabrication d'une forme galénique, contenant une composition pharmaceutique selon une ou plusieurs des revendications 1 à 9, **caractérisé en ce qu'**on prépare une composition pharmaceutique selon la revendication 10 ou 11, on la transforme en granulés, granules ou poudres, éventuellement on la formule au moyen d'adjuvants pharmaceutiquement usuels et on la transforme d'une façon connue, par exemple par mélange, compression, powder layering et/ou encapsulation en une forme galénique, par exemple en comprimés, ou de préférence en une forme galénique multiparticulaire, en particulier en comprimés contenant des granules, minicomprimés, gélules, sachets ou poudres solubles.

13. Forme galénique, contenant une composition pharmaceutique selon une ou plusieurs des revendications 1 à 9.

14. Utilisation d'une composition pharmaceutique selon une ou plusieurs des revendications 1 à 9, pour la fabrication d'une forme galénique.
